# EUROPEAN PATENT SPECIFICATION

(11) **EP 1 603 595 B1**
(45) Date of publication and mention of the grant of the patent: **10.11.2010**
(21) Application number: 04714961.2
(22) Date of filing: 26.02.2004
(51) Int. Cl.: A61K 45/00, A61K 31/4178, A61K 31/445, A61K 31/55, A61P 25/30, A61P 25/32, A61P 25/36

(54) **PHARMACEUTICAL COMPOSITION FOR TREATMENT OF DRUG DEPENDENCE**
PHARMAZEUTISCHE ZUSAMMENSETZUNG ZUR BEHANDLUNG VON DROGENABHÄNGIGKEIT
COMPOSITION PHARMACEUTIQUE POUR LE TRAITEMENT DE LA PHARMACODEPENDANCE

(30) Priority: 27.02.2003 US 449868 P
(43) Date of publication of application: 14.12.2005
(73) Proprietor: Eisai R&D Management Co., Ltd., Tokyo 112-8088 (JP)
(72) Inventor: NAKANISHI, Shigetada, Kyoto-shi, Kyoto 606-0042 (JP); HIKIDA, Takatoshi, Baltimore, Maryland 21209-4942 (US)
(74) Representative: von Kreisler Selting Werner
(86) International application number: PCT/JP2004/002301
(87) International publication number: WO 2004/075916

(56) References cited:
- EP-A2- 0 515 301
- WO-A1-94/16708
- WO-A1-95/29909
- WO-A1-96/29332
- WO-A1-99/07359
- WO-A1-99/07363
- WO-A1-02/085370
- WO-A2-00/09483
- AU-B2- 738 033
- JP-A- 11 171 861
- WONG CHAK-LAM ET AL: 'Further studies on the role of cholinergic mechanisms in the development of increased naloxone potency in mice' EUROPEAN JOURNAL OF PHARMACOLOGY vol. 56, no. 1-2, 1979, pages 115 - 121, XP002980170
- DATABASE CAPLUS 1984 XP002980171 Retrieved from STN Database accession no. (106222)
- BOROWSKI T.B. ET AL: 'The effects of tacrine on cocaine self-administration in rats: a potential pharmacotherapy for cocaine addiction' SOCIETY FOR NEUROSCIENCE vol. 26, no. 1-2, 2000, XP002980172
- MAJEWSKA M.D. ET AL: 'a maverick agent in cocaine dependence' SOCIETY FOR NEUROSCIENCE ABSTRACTS vol. 26, no. 1-2, 2000, XP002980173
- HIKIDA T. ET AL: 'Acetylcholine enhancement in the nucleus accumbens prevents addictive behaviors of cocaine and morphine' PROCEEDINGS OF THE NATIONAL ACADEMY OF SCIENCES OF THE UNITED STATES OF AMERICA vol. 100, no. 10, 2003, pages 6169 - 6173, XP002980174

## Description

### TECHNICAL FIELD

The present invention relates to the treatment of drug dependence, more specifically, to a pharmaceutical composition for the treatment of drug dependence, which comprises a medicinal substance capable of enhancing or disinhibiting the action of acetylcholine, a neurotransmitter, in the central nervous system (CNS) and a method of treating drug dependence with such a substance.

### BACKGROUND ART

Drug- or substance-dependence is a disorder caused by various types of addictive (dependence-producing) substances. The number of patients/drug abusers suffering from drug dependence is estimated to exceed 30 millions worldwide. The cost to society is high when social problems caused by drug-dependence-related organic mental disorder and socially dysfunctional characteristics of drug dependent individuals are considered. Thus there is a worldwide demand for an effective cure for drug dependence.

There are two types of drug (substance) dependencies; psychological and physical. Psychological dependence refers to a condition wherein an organism possesses a heightened desire and compulsion for taking a certain drug. Physical dependence refers to a condition wherein both psychologically and physically morbid symptoms (withdrawal symptoms) occurs to an organism when the drug is depleted from the body and the pharmacological effect thereof weakens or vanishes after having habituated to the condition under the influence of an addictive drug.

To the onset of drug dependence, the involvement of the CNS reward system has been elucidated. The reward system has been identified as the site responsible for intracranial self stimulation-related behaviors in animals and plays a role in eliciting senses of pleasure, motivation, and euphoria. The treatment of drug dependence is made very difficult since many addictive substances have an activity of stimulating this system, thereby eliciting senses of pleasure in users, and the influence of such activity remains even after the drug, as a causative agent, is depleted from the body. In accordance with the symptoms, addictive drugs/substances are classified into the following types: morphine type, alcohol type, barbiturate type, amphetamine type, cocaine type, cannabinoid type, organic solvent type, khat type, and hallucinogen type. Examples of substances generally known to cause drug dependence include cocaine, opium (heroin, morphine, etc), alcohols, amphetamine (or amphetamine-like substances), caffeine, cannabinoids, hallucinogen, inhalants, nicotine, phencyclidine (or phencyclidine-like substances), sedatives, hypnotic agents and anxiolytic agents. Patients are often found to be dependent on more than one type of these substances.

Most addictive drugs can cause dependence after a single administration and once the user is affected, the symptoms sometimes persist over a long term even after the use is terminated. For these reasons, drug dependence is considered as a chronical neurological disorder. Furthermore, overdose of such drugs may have a deteriorating effect on living body and may even cause death.

Throughout the present specification and claims, the term "drug dependence" means conditions including "substance-related disorders" based on the criterion of DSM-IV (Diagnostic and Statistical Manual of Mental Disorders, Fourth Edition) of American Psychiatric Association, namely, substance use disorders and substance-induced disorders.

At present, there are few effective cures for drug dependence. Cocaine- or amphetamine-dependence is currently treated by psychotherapy, however, its effects are poor, and there is no effective medical therapy available. Treatment with dopamine antagonists or agonists is considered, however, there is little therapeutic effect. Psychotic disorders induced by cocaine or amphetamine are treated by symptomatic treatment with antipsychotic drugs. Opium or nicotine dependence is treated by alternative substances such as methadone or nicotine patch. Although such therapy may be effective on withdrawal symptom, the dependence itself remains. Regarding benzodiazepine, it shows certain effects on withdrawal symptoms of alcohol dependence, but it is ineffective on alcohol dependence per se. An alcohol deterrent disulfiram is useful in the rehabilitation period of alcohol dependence treatment but may cause noxious reaction when taken simultaneously with alcohol. Therefore, the administration of disulfiram must be combined with psychological or behavioral therapy, and hence causes practical inconvenience. An opioid receptor antagonist, naltrexone, has been approved in the U.S.A. as a therapeutic medicament, however, the effects of this drug is yet to be established.

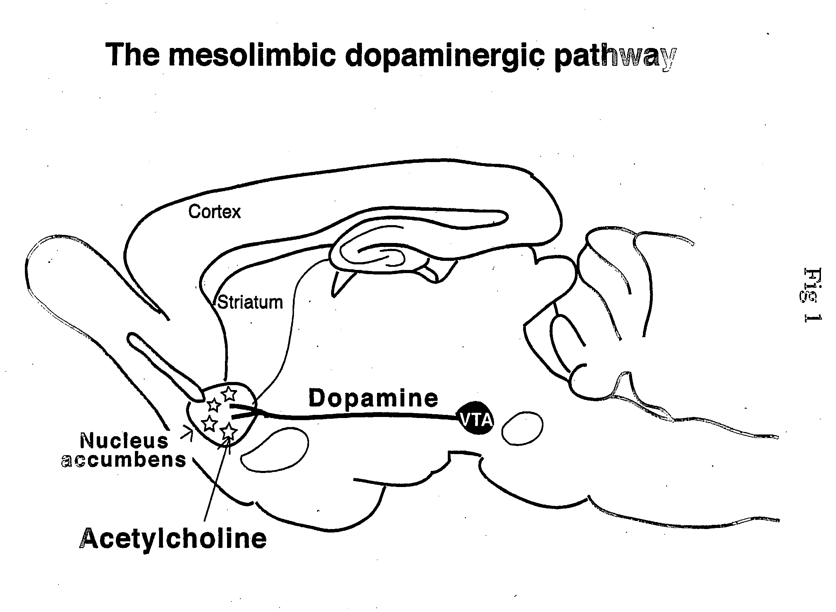

The regions of the brain that are closely associated with the reward system, the system closely related to drug dependence, include; ventral tegmental area (hereinafter, "VTA"), nucleus accumbens (NAc), locus ceruleus and medial forebrain bundle. In particular, the dopaminergic system projecting from VTA to NAc plays the central role in the activation of the reward system (Fig. 1). Fig. 1 illustrates the dopaminergic pathway of the mesolimbic system. As seen from Fig. 1, the reward system functions when dopamine derived from VTA acts on nerve cells of NAc. Cocaine increases the NAc dopamine level through inhibition of dopamine transporters of nerve cells in the NAc. It is also known that opium, amphetamine and alcohols elevate the NAc dopamine level. Therefore, increase of dopamine level in NAc is implicated in inductive mechanism of drug dependence.

BOROWSKI T.B. ET AL: 'The effects of tacrine on cocaine self-administration in rats: a potential pharmacotherapy for cocaine addiction' SOCIETY FOR NEUROSCIENCE vol. 26, no. 1-2, 2000 discloses that the acetylcholinesterase inhibitor tacrine blocked dose-dependently cocaine self-administration and is therefore considered as useful for the treatment of cocaine addiction.

WO-A1-94/16708 discloses the use of galanthamin for the treatment of nicotine dependence.

From this viewpoint, the researches of this field were focused on drugs that target dopamine itself or other neurotransmitters indirectly related to the dopaminergic nervous system such as serotonin, GABA or glutamic acid, receptors thereof and intracellular signaling cascades associated with dopamine receptors. However, no investigation of such kind has resulted in the development of any substance effective for the treatment of drug dependence so far.

NAc contains cholinergic cells that produce acetylcholine (ACh), which is another neurotransmitter different from dopamine. The present inventors have established a method to selectively eliminate cholinergic cells in the NAc, and demonstrated that ACh has an antagonistic activity against dopamine activities and that the reduction of NAc ACh level by cholinergic cell elimination enhances the sensitivity to cocaine in mice (Hikida et al., Proc. Natl Acad. Sci. Use 98, 13351-13354 (2001)).

### DISCLOSURE OF INVENTION

The present inventors, with consideration to the situation above, have conducted an investigation with a purpose of developing medicaments effective for the treatment of drug dependence. They approached from perspectives different to those from which many researchers have so far attempted. The present inventors focused on the relationship between the NAc ACh level and the onset of drug dependence. As a result of an extensive investigation, it was found that a substance capable of enhancing or disinhibiting the action of ACh is useful in the treatment of drug dependence.

A first embodiment of the present invention is a pharmaceutical composition for use in the treatment of drug dependence which comprises as an active ingredient donepezil or its salt.

Preferred embodiments are referred to in dependent claims 2 to 5.

A further embodiment of the invention is the use of donepezil or its salt for the preparation of a therapeutic agent for treating drug dependence.

Preferred embodiments are referred to in dependent claims 7 to 10.

A further embodiment is donepezil or its salt for use in the treatment of drug dependence.

Preferred embodiments are referred to in claims 12 to 15.

### BRIEF DESCRIPTION OF DRAWINGS

Fig. 1 is a schematic drawing of the NAc in the mesolimbic system. The NAc is a key neural substrate responsible for drug dependence. The neural activity of the NAc is controlled by not only dopamine derived from VTA but also ACh released from cholinergic cells within the NAc.

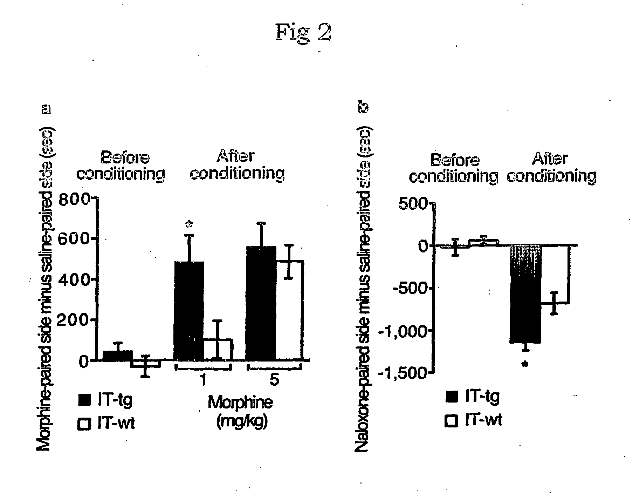

Fig. 2 shows the results obtained in Experiment 1, wherein the reduction of ACh level by cholinergic cell elimination in the NAc was examined on the sensitivity to an addictive drug. In the figure, "IT" refers to immunotoxin (IT), "IT-tg" refers to cholinergic cell-eliminated (ablated) mice and "IT-wt" refers to IT-injected wild-type mice used as control. The IT-tg mouse was a transgenic mouse wherein cholinergic cells in the NAc were selectively eliminated by IT injection according to the immunotoxin-mediated cell targeting (IMCT) technique. Fig. 2a shows the results of conditioned place preference ("CPP") test. The test was conducted by first allowing mice to associatively learn the relationship between the rewarding effect of morphine and the environmental cues of the drug-paired chamber through administering morphine at one fixed chamber and saline at the other fixed chamber, and then measuring the time mice spent in respective chambers while mice were allowed to move freely after the conditioning procedure. CPP is expressed in the difference in time (seconds) spent in the respective chambers, i.e., the time spent in morphine-paired chamber was subtracted from the time spent in saline-paired chamber (vertical axis). Fig. 2b shows the results of conditioned place aversion (CPA) test, wherein, after the establishment of morphine dependence, mice were place-conditioned by receiving saline or naloxone, an opioid receptor antagonist, following an administration of morphine, thus causing withdrawal symptoms of morphine dependence. CPA is also expressed by the time difference (seconds), in which the time mice spent in the naloxone-paired chamber was subtracted from the time mice spent in the saline-paired chamber (vertical axis) while mice were allowed to move freely.

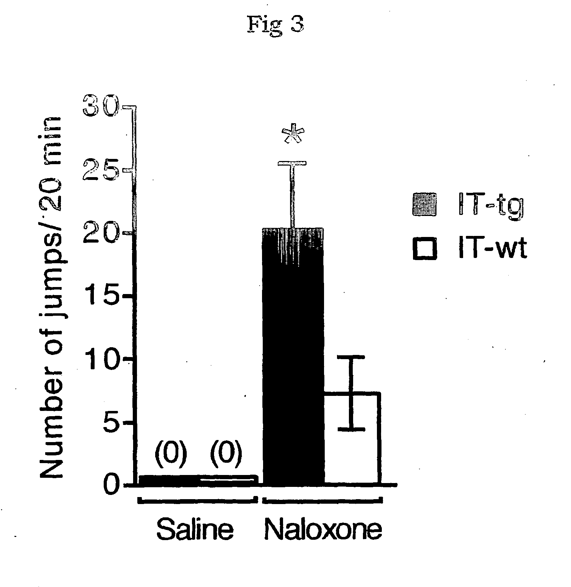

Fig. 3 shows the results obtained in Experiment 2, wherein the effect of the NAc ACh level reduction on the physical dependence to an addictive drug was examined in cholinergic cell-eliminated and wild-type mice. As is the case with Fig. 2, IT-tg and IT-wt mice were made physically depend on morphine. The mice then received naloxone or saline following a morphine administration, and the number of expressed withdrawal symptoms (jumps) was counted.

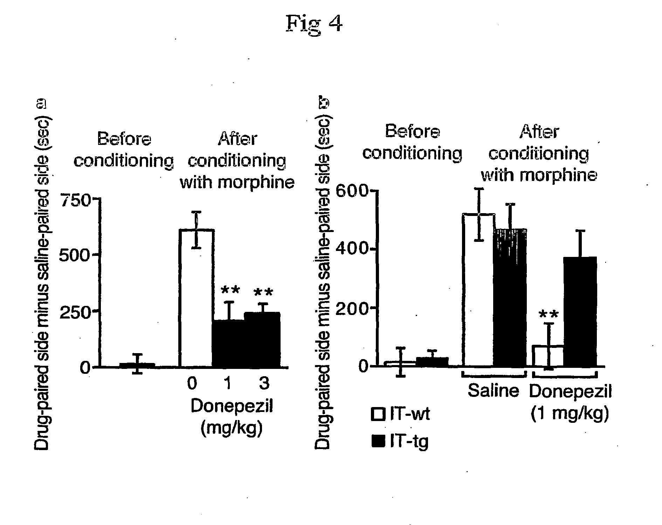

Fig. 4 shows the results obtained in Example 1, wherein the effects of a ChE inhibitor on psychological morphine dependence were examined by CPP test. Fig. 4a shows the effects of pretreatment with donepezil before the administration of morphine on the place preference of wild-type mice in the CPP test. Fig. 4b shows the effects of pretreatment with donepezil or saline before the administration of morphine on the place preference of IT-tg or IT-wt mice.

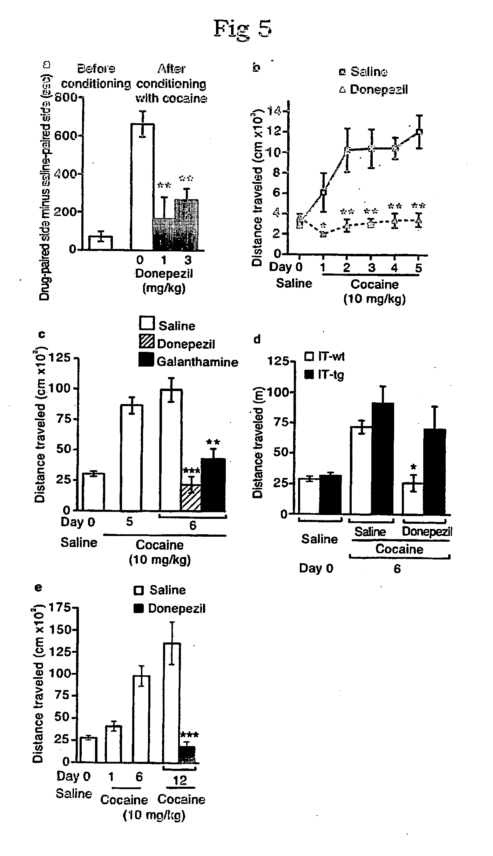

Fig. 5 shows the results obtained in Example 2 conducted using cocaine. As in Fig. 4a, Fig. 5a shows the effects of donepezil on cocaine dependence in wild-type mice, using CPP test. Fig. 5b shows the effects of pretreatment with donepezil on hyperlocomotion (locomotor sensitization) induced by repeated administration of cocaine; the distance traveled (cm x 10²) was measured as an indicator of changes in the locomotor activity. Fig. 5c shows the effects of donepezil and galanthamine on cocaine-induced hyperlocomotion, expressed in distance traveled, in wild-type mice. Fig. 5d shows hyperlocomotion (distance traveled) induced by repeated administration of cocaine in IT-tg and IT-wt mice. Fig. 5e shows the effects of donepezil on relapse of cocaine-induced hyperlocomotion in wild-type mice which were initially sensitized with repeated cocaine administration and then re-administered with cocaine after a long-term drug deprivation.

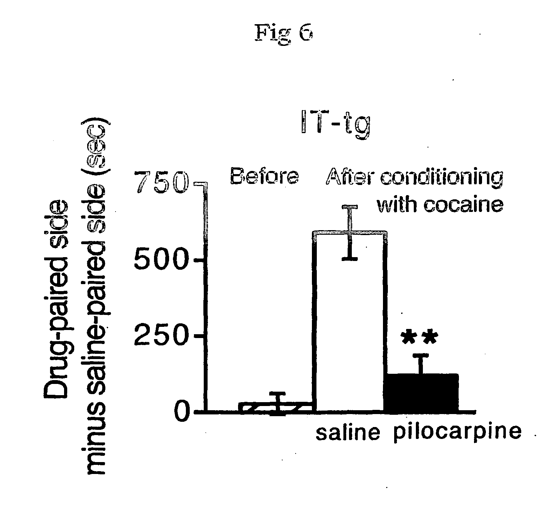

Fig. 6 shows the results obtained in Example 3, wherein the effects of pilocarpine, which is a muscarinic ACh receptor agonist, on psychological cocaine dependence in IT-tg mice was examined by CPP test.

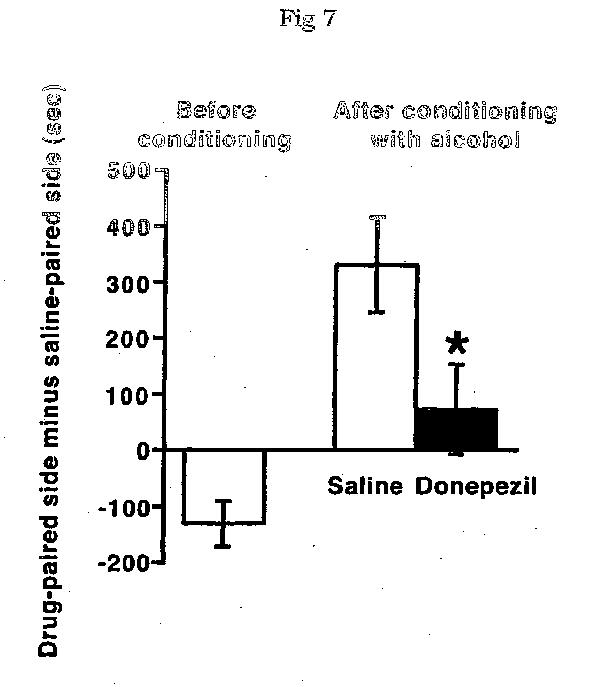

Fig. 7 shows the results obtained in Example 4, wherein the effects of donepezil on alcohol dependence in wild-type mice were examined by CPP test.

### BEST MODE FOR CARRYING OUT THE INVENTION

The present inventors for the first time found that enhancement or disinhibition of the cholinergic nervous system in the NAc is effective in treating drug dependence as will be hereinafter described through Experiments and Examples below.

The pharmaceutical composition of the present invention is effective on drug dependence associated with symptoms of both psychological and/or physical dependence as well as sensitization (reverse tolerance), and hence is applicable to a wide range of drug dependence symptoms.

The pharmaceutical composition of the present invention comprises as an active ingredient, donepezil or its salt.

Throughout the present specification and claims, the term "drug dependence" has the same meaning as the one generally used in the field in which the present invention pertains, and means a condition where a drug user is psychologically and/or physically dependent on a certain drug/ substance. Psychological dependence refers to a condition where one's desire of using an addictive drug is so strong that it cannot be suppressed by will. Physical dependence refers to a condition where one exhibits various symptoms of drug addiction and develops withdrawal or abstinence symptoms upon reduction or withdrawal of addictive drugs. These symptoms include insomnia, anxiety, convulsive seizure, hallucination and so forth.

According to the causative drug, drug dependence is generally classified into morphine type, alcohol type, barbiturate type, amphetamine type, cocaine type, cannabinoid type, organic solvent type, khat type and hallucinogen type.

The present pharmaceutical composition is useful for the treatment of dependence caused by any addictive drug; however, it is preferably used for dependence caused by a drug of morphine type, alcohol type, barbiturate type or cocaine type, more preferably morphine, cocaine or alcohol, in order to obtain excellent effects.

The term "a medicinal substance having an activity of enhancing or disinhibiting the action of ACh" refers to medicinal substances capable of enhancing or disinhibiting actions of ACh. These are collectively referred to as cholinergic agent. Examples of cholinergic agent include (1) cholinergic alkaloids, (2) ChE inhibitors, and (3) cholinesters (e.g., a substance that acts on muscarinic receptor or nicotinic receptor such as ACh, methacholine, carbachol, bethanechol, etc.). For the present invention, donezepil or its salt is used since it can cross the blood brain barrier, and is active in the nervous system. The ChE inhibitor is donezepil or its salt.

For the present invention, any available donepezil, such as donepezil or its salt is effective. Those compounds and a method of preparing them are known and are described in JP-1171861A/1999, JP 2002-525264 A.

When ChE inhibitors are used which are derivatives that are functionally analogous to galanthamine, they are defined herein as compounds which possess an at least 10-fold selectivity, preferably an at least 20-fold selectivity, more preferably an at least 40-fold selectivity, and most preferably an at least 50-fold more selectivity for acetylcholinesterase than for butylcholinesterase, when evaluated by the in vitro method of the following references (Thomsen and Kewitz, Life Sciences 46, 1553-1558 (1990); Thomsen et al., J. Clin. Chem. Clin. Biochem. 26, 469-475 (1988)).

For example, galanthamine hydrobromide possess 50-fold more selectivity when examined under the conditions described by Thomsen and Kewitz (Life Sciences 46, 1553-1558 (1990)). Accordingly, whether a candidate substance has an activity of "enhancing or disinhibiting the actions of ACh" can be evaluated by in vitro method of Thomsen et al. as described in the example of galanthamine hydrobromide above. The candidate substance can be a medicinal substance if it possesses an at least 10-fold selectivity, preferably an at least 20-fold selectivity, more preferably an at least 40-fold selectivity, and most preferably an at least 50-fold more selectivity for acetylcholinesterase than for butylcholinesterase.

When a ChE inhibitor used in the present invention is donepezil or its salt, such ChE inhibitor will possess an at least 10-fold more selectivity, preferably 100-fold more selectivity, and more preferably 1000-fold more selectivity for acetylcholinesterase than for butylcholinesterase.

The present pharmaceutical composition can be formulated into a form suited for oral or parenteral administration in a conventional manner by admixing donepezil, or a salt thereof, an active ingredient with one or more pharmaceutically acceptable carriers, excipients or diluents, as required. The present composition may be in any form depending on the selected administration route (oral administration, intravenous, intramuscular or subcutaneous injection, or intrathecal administration via implanted device), for example, tablets, powder, granules, capsules, solutions, lozenges, pessaries, creams, suppositories, transdermal formulations such as patch, cream, ointment or lotion. These or other formulations can be prepared by a method known in the art.

For example, tablets may be prepared by subjecting an active ingredient and an additive(s) to compression or tablet compression. Examples of additives that may be used in the present invention include pharmaceutically acceptable excipients such as binders (e.g., corn starch), fillers (e.g., lactose, microcrystalline cellulose), disintegrants (e.g., sodium starch glycolate) or lubricants (e.g., magnesium stearate). The tablets may be coated in a conventional manner. When the preparation is a liquid preparation such as syrup, solution, or suspension, it can be prepared using, for example, a suspending agent (e.g., methylcellulose), an emulsifier (e.g., lecithin), and/or a preservative. As for injectable preparations, they may be in the form of solution, suspension, W/O or O/W emulsion, which preparations may further contain a suspension stabilizer or a dispersant. When the preparation is used as an inhalant, it is formulated as a liquid preparation applicable to an inhaler.

The pharmaceutical composition of the present invention is preferably in the form suited for oral and transdermal administration routes with consideration to the convenience of preservation, transportation, and patient compliance.

The dosage of a cholinergic agent varies depending upon the kind of drug(s) selected, the mode of administration, the condition, age, weight, sex of the patient, and the concomitant drug if any. However, the ultimate decision is made by the medical practitioner based on the individual circumstances. For oral administration, the daily dosage may be within the range of 2-100 mg, preferably 5-70 mg, more preferably 10-30 mg. For parenteral administration, the daily dosage may be within the range of 0.1-100 mg, preferably 5-100 mg, more preferably 10-50 mg, and further preferably 5-30 mg. It is often preferred to start with lower dosages. The dosage for transdermal administration of a medicinal substance such as galanthamine is similar to that of oral administration.

The present composition may contain one or more cholinergic agents, and, if necessary, other concomitant drug(s). Examples of drugs usable in combination include dopamine receptor antagonists such as haloperidol. The concomitant drugs may be formulated together with the cholinergic agent of the present invention in a single preparation or may be formulated as a separate preparation and administered simultaneously or non-simultaneously with the present pharmaceutical composition.

When treating drug dependence according to the present invention in a patient in need thereof, the pharmaceutical composition obtained above is administered to the patient through an appropriate route and method. The method for administration is known to those skilled in the art.

The present invention will be hereinafter described in more detail by referring to examples.

### EXPERIMENT 1: Test of Sensitivity to Psychological Morphine Dependence in NAc Cholinergic Cell-eliminated Mice

### Preparation of Cholinergic cell-eliminated mouse (IT-tg mouse)

Cholinergic cell-eliminated (IT-tg) mice wherein ACh-expressing cells (cholinergic cells) in the NAc were ablated were prepared and used in the experiment for examining the effects of reduction of ACh level in the NAc on sensitivity to an addictive drug (a drug dependence-producing drug). Selective ablation of cholinergic cells in the NAc was conducted through immunotoxin-mediated cell targeting (IMCT) techniques using transgenic mice and anti-Tac(Fv)-PE38 as the immunotoxin (Kobayashi et al., Proc. Natl Acad. Sci. USA 92, 1132-1136 (1995); Watanabe et al., Cell 95, 17-27 (1998)). Anti-Tac (Fv)-PE38 is a recombinant fusion protein composed of a variable region of a monoclonal antibody against human interleukin 2 receptor α subunit (hIL-2Rα) and Pseudomonas exotoxin, and can be purified from E. coli BL21 as described by Chaudhary et al. (Nature 339, 394-397 (1989)). Anti-Tac(Fv)-PE38 is an immunotoxin that can specifically recognize cells expressing hIL-2Pα and inhibits their protein synthesis, thereby eliminating the cells expressing hIL-2Rα. The present inventors have developed a method for selectively ablating cholinergic cells in the NAc; the method comprising administering anti-Tac(Fv)-PE38 locally to a transgenic mouse whose NAc specifically expressed hIL-2Rα in cholinergic cells. In the NAc, only cholinergic cells express metabotropic glutamate receptor subtype 2 (mGluR2). Accordingly, the present inventors have produced a transgenic mouse expressing a fusion hIL-2Rα/GFP protein composed of hIL-2Rα and green fluorescent protein (GFP) under the control of a 18.3 kbp upstream fragment of mouse mGluR2 gene as a promoter, as described by Watanabe et al. (Cell 95, 17-27 (1998)). Both wild-type and transgenic mice were C57B6/J strain. The preparation of IT-tg was conducted by stereotaxically injecting a solution of IT (10 ng) in 0.5 µl phosphate-buffered saline into the NAc of transgenic mice (9-13 weeks) over 3 minutes from the tip of a glass needle placed at the point 1.5 mm anterior and 0.8 mm lateral from bregma of the skull at the depth of 3.5mm (Hikida et al., Proc. Natl Acad. Sci. USA 98, 13351-13354 (2001)). Immunostaining analysis with an antibody against choline acetyltransferase that is responsible for ACh synthesis in the brain revealed that more than 70% of cholinergic cells were eliminated from the NAc in transgenic mice two weeks after IT injection. Cell ablation with IT was limited to ACh-expressing cells, and no reduction in the numbers of dopaminergic cells, GABA-positive cells, or parvalbumin-positive cells in NAc was observed. In addition, the reduction of the ACh level to 23% of the normal level was achieved without affecting the level of dopamine and its metabolites, i.e., 3,4-dihydroxyphenylacetic acid (DOPAC) and homovanilic acid (HVA) (Kaneko et al., Science 289, 633-637 (2000)). The above-mentioned IT-treated transgenic mouse was used as cholinergic cell-eliminated mouse (IT-tg) in the Experiments and Examples hereinafter described.

The IT per se is known not to eliminate the cholinergic cells of wild-type mice (Hikida et al., Proc. Natl Acad. Sci. USA 98, 13351-13354 (2001)). Both wild-type and transgenic mice received bilateral IT injection into the NAc.

### A. Conditioned Place Preference Test

Using the IT-tg and IT-wt mice, the effect of reduction of NAc ACh level on the sensitivity to an addictive drug was analyzed by examining their behavior.

Anti-Tac(Fv)-PE38 (IT) was injected into the NAc of mice, and two weeks later, both IT-tg and IT-wt mice were subjected to the conditioned place preference (CPP) test using morphine. The CPP test makes it possible to quantify the degree of psychological dependence on an addictive drug. That is, when an addictive drug is administered to animals in a given environment, these animals associate the drug to the environmental cues and tend to spend longer time in the drug-associated environment ("acquire the place preference"). For example, the CPP test can be carried out by the method of Kelz et al. (Nature 401, 272-276 (1999)).

The CPP test was carried out in a three-chambered apparatus consisting of a small middle chamber that connected two large chambers on either side, wherein the two large chambers differed in the floor and wall patterns. Before the conditioning (day 0), mice were allowed to move freely across the three chambers for 30 min. Then, for the following 3 days, mice were conditioned as follows. Two doors connecting the three chambers were shut to separate the chambers and mice were confined to one large chamber for 20 min immediately after intraperitoneal injection of physiological saline. Four hours later, mice received intraperitoneal injection with 1 mg/kg or 5 mg/kg of morphine hydrochloride, and were confined to the other large chamber for 20 min. After the 3-day-conditioning procedure, mice were placed in the middle small chamber with the doors open and they were allowed to move freely across the three chambers for 30 min. The time (seconds) mice spent in each of the large chambers was measured with an infrared apparatus. Psychological morphine dependence of IT-tg mice and IT-wt mice was quantified by calculating the difference in the time mice spent in each chamber through subtracting the time spent in the saline-paired chamber from the time spent in the morphine-paired chamber. (IT-tg: n=8-10; IT-wt: n=7-12) The results of the present and following behavioral experiments are provided in the histogram or sequential-line graph as shown in the drawings attached hereto. The mean value of respective group is depicted together with the error bar showing the standard error. Statistical analysis was conducted by ANOVA (analysis of variance) and multiple comparisons were made with Scheffe's test.

The difference in time spent in the morphine-paired side and the saline-paired side (morphine-paired side minus saline-paired side) was calculated and is depicted on the vertical axis. Before the conditioning, both IT-tg and IT-wt mice showed no preference in visiting either places (Fig. 2a, before conditioning). It can be seen from Fig. 2a that, after the 3-day-conditioning with 1 mg/kg morphine, IT-tg mice exhibited a significant preference to visiting the morphine-paired chamber, while no such preference was observed in It-wt mice (Fig. 2a, after conditioning, *, P<0.05). When conditioned with 5 mg/kg morphine, both IT-tg and IT-wt mice exhibited place preference. Accordingly, it has been revealed that the reduction of ACh level in NAc by cholinergic cell elimination results in the elevation of sensitivity to morphine-dependence in CPP test.

In a similar experiment, CPP test was carried out using cocaine instead of morphine. In such experiment, It-tg mice showed stronger preference for the cocaine-paired chamber than IT-wt mice after 3 days of conditioning with 5 mg/kg or 10 mg/kg cocaine (P<0.01, Hikida et al., Proc. Natl Acad. Sci. USA 98, 13351-13354 (2001)).

These results indicate that the reduction of ACh level in NAc elevates the sensitivity to an addictive drug such as morphine and cocaine.

### B. Conditioned Place Aversion Test

When drug administration is suspended from a morphine dependent condition, discomfort occurs with withdrawal symptoms. The effect of the NAc ACh level reduction on the negative reinforcement of morphine, the withdrawal symptom, was examined by conditioned place aversion (CPA) test. CPA test can be carried out by a method of, for example, Murtra et al. (Nature 405, 180-183 (2000)). Morphine dependence was established in mice by twice daily intraperitoneal administrations of morphine with a gradual increment (10 mg/kg each) from 10 to 40 mg/kg morphine over 4 days (1-4 days). On day 5, mice were place-conditioned with the same apparatus as mentioned in "A" above. That is, mice received saline 1 hour after the administration of 50 mg/ml morphine, and were immediately confined to one of the two large chambers for 20 min. Four hours later, mice received intraperitoneal injection with 50 mg/kg morphine, followed by 1 mg/kg of naloxone, a morphine antagonist, and they were immediately confined to the other large chamber for 20 min. On the following day, mice were placed in the small middle chamber with doors open so that they can move freely across the three chambers for 30 min. The time (seconds) mice spent in the large chambers was measured with an infrared apparatus. The effect of the aversive stimuli, the negative reinforcement caused by morphine withdrawal, on IT-tg mice and IT-wt mice was quantified by calculating the difference in the time mice spent in the two chambers, in which the time spent in the saline-paired chamber was subtracted from the time spent in the naloxone-paired chamber (naloxone-paired side minus saline-paired side; n=8 for each group). Before the conditioning, both IT-tg and IT-wt mice showed no preference over the two chambers (Fig. 2b, before conditioning). After the conditioning, both IT-tg and IT-wt mice exhibited place aversion to the chamber, which was paired to drug deprivation through the conditioning with naloxone; however, IT-tg mice showed stronger place aversion (Fig. 2b, after conditioning, *, P<0.05). It has been proved that the reduction of ACh level in NAc enhances the effect of negative reinforcement caused by morphine withdrawal. The results above indicate that a medicinal substance capable of preventing ACh level reduction is useful in alleviating the discomfort caused by morphine deprivation.

### EXPERIMENT 2 Morphine Withdrawal Symptom in Cholinergic Cell-eliminated Mice

Physical morphine dependence develops after chronic morphine administration. The morphine withdrawal symptoms due to morphine withdrawal hamper the treatment of drug dependence. The effect of NAc ACh level reduction on physical dependence was analyzed by examining the degree of withdrawal symptoms induced by morphine deprivation using naloxone. Morphine dependence was established in mice as described in Experiment 1B. On day 5, either saline or naloxone (1 mg/kg) was injected 1 hr after the morphine (50 mg/kg) administration. Jumping, a physical morphine withdrawal symptom, was then counted over 20 min (n=8 for each group). This symptom was observed in both IT-tg mice and IT-wt mice (control) that were treated with naloxone. However,. IT-tg mice exhibited a significantly more number of jumps when compared to IT-wt mice (Fig. 3, *, P < 0.05). It has been revealed that the reduction of ACh level in NAc enhances the morphine withdrawal symptoms. The above results indicate that a medicinal substance capable of preventing reduction of ACh level in NAc is useful in alleviation of morphine withdrawal symptoms.

### EXAMPLE 1 The Effects of Inhibiting the ACh Level decrease on Psychological Morphine Dependence

### A. CPP Test in Wild-type Mice

Experiment 1A has shown that the reduction of ACh level in NAc enhances the sensitivity to morphine. In the present Example, whether morphine dependence can be treated by preventing the reduction of ACh level with donepezil, a ChE inhibitor, was examined. Wild-type mice (9-13 weeks) were place-conditioned by daily administration of morphine (5 mg/kg) over 3 days as described in EXPERIMENT 1A. Mice received intraperitoneal injection with saline or donepezil hydrochloride (1 or 3 mg/kg) 20 minutes before the administration of morphine in the place-conditioning procedure (n=7-11, for each group). Pretreatment with donepezil of both doses significantly reduced the development of morphine-induced CPP (Fig. 4a, **, P < 0.01). It has been revealed that the inhibition of reduction of ACh level with donepezil can alleviate psychological morphine dependence. These results indicate that a medicinal substance capable of enhancing or disinhibiting ACh actions is useful in the treatment of morphine dependence.

### B. CPP Test in Cholinergic Cell-eliminated Mice

The effect of inhibiting the ACh level reduction on psychological morphine dependence was examined by comparing IT-tg mice with IT-wt mice (control). Mice received intraperitoneal injection with saline or donepezil hydrochloride (1 mg/kg) 20 min prior to each administration of morphine (5 mg/kg) in a 3-day place-conditioning procedure. (n = 7-9). Donepezil significantly reduced morphine-induced CPP in IT-wt mice (Fig. 4b, **, P < 0.01) but failed to suppress morphine-induced CPP in IT-tg mice (Fig. 4b). The above results indicate that donepezil alleviate psychological morphine dependence through the inhibition of degradation of ACh produced by cholinergic cells in the NAc. Thus, a medicinal substance capable of enhancing or disinhibiting the action of ACh derived from cholinergic cells in the NAc can markedly alleviate the psychological morphine dependence.

### EXAMPLE 2 Effects of Inhibiting the ACh Level Reduction on Cocaine Dependence

### A. Preventative Effects of Donepezil on Cocaine dependence

It has been proved that the reduction of ACh level in the NAc by cholinergic cell elimination enhances cocaine dependence (Hikida et al., Proc. Natl Acad. Sci. USA 98, 13351-13354 (2001)). In the present Example, CPP test was conducted to examine whether inhibiting the reduction of ACh level has a potential for treating cocaine-induced drug dependence. As described in Example 1A, wild-type mice (9-13 weeks) were place-conditioned over 3 days through daily administration of cocaine (10 mg/kg). Mice received intraperitoneal injection with saline or donepezil hydrochloride (1 or 3 mg/kg) 20 min prior to the administration of morphine in the place-conditioning procedure (n=7 to 11, for each group). Pretreatment with donepezil of both doses significantly reduced the degree of cocaine-induced CPP (Fig. 5a, **, P < 0.01). These results indicate that the inhibition of reduction of the NAc ACh level can alleviate psychological cocaine dependence. Thus, it has been demonstrated that a medicinal substance capable of enhancing or disinhibiting ACh actions can be useful in the treatment of psychological cocaine dependence.

### B. Preventative Effects of Donepezil on Cocaine-induced Sensitization

Repeated cocaine administration induces a progressive increase in locomotor activity (hyperlocomotion), which condition is called "locomotor sensitization" (Hikida et al., Proc. Natl Acad. Sci. USA 98, 13351-13354 (2001); Koob, Neuron 16, 893-896 (1996)). The drug-induced sensitization is one of significant mechanisms contributing to the development of drug dependence. IT-tg mice showed greater increase in the locomotor activity by daily cocaine administration compared to IT-wt mice (Hikida et al., Proc. Natl. Acad. Sci. USA, 98, 13351-13354 (2001)). Thus, the decrease in ACh level enhances the symptoms of drug-induced sensitization. In the present EXAMPLE, whether inhibiting the reduction of the ACh level can prevent the development of locomotor sensitization was examined.

Wild-type mice (9-13 weeks) were put into a locomotor activity-measuring chamber for 3 days to habituate. Then onwards, immediately after the daily intraperitoneal injection of cocaine (10mg/kg), mice were put into the chamber for 10 minutes and their locomotor activity (the distance moved) was measured using an infrared apparatus. The activity was later converted into the distance traveled. Ten minutes prior to the administration of cocaine, mice were either administered with saline or 1mg/kg of donepezil (n=10 for each group). In the groups pretreated with saline, a significant increase in locomotor activity was observed while no such increase was observed in the group pre-treated with donepezil (fig. 5b). According to statistical analysis by repeated analysis of variance (ANOVA), the pretreatment resulted in a significant difference (F = 20.7, ***P < 0.001). When the comparison analysis was conducted on each day of the cocaine administration, the following significant differences were obtained: *P < 0.05 for day 1, and **P < 0.01 for days 2-5. Thus, the development of sensitization by chronic cocaine administration was prevented by pretreatment with donepezil. These results indicate that the inhibition of reduction of ACh level is effective in suppressing the development of cocaine dependence. These results also demonstrate that a medicinal substance capable of enhancing or disinhibiting ACh actions has an ability of preventing drug dependence.

### C. Therapeutic Effects of Donepezil (according to the invention) and Galanthamine (not according to the invention) on Cocaine Dependence

The present investigation was conducted to examine whether inhibiting the ACh level reduction has a potential for treating cocaine-induced drug dependence after its establishment. Wild-type mice (9-13 weeks) received intraperitoneal injection of cocaine (10 mg/kg) once a day for 5 days. As a result, the mice exhibited increased locomotor activity and served as a model of cocaine dependence in the experiment. On day 6, the mice were pretreated with saline, donepezil (1 mg/kg) or galanthamine (1 mg/kg) 10 min before intraperitoneal injection of cocaine (10 mg/kg), and the cocaine-induced locomotor activity was measured for 10 min (n = 5-12 for each group). Pretreatment with donepezil or galanthamine, another kind of ChE inhibitor, was significantly effective in inhibiting cocaine-induced hyperlocomotion observed on day 6 (Fig. 5c, ***, P < 0.001, **, P < 0.01). These results show that cocaine-induced chronic abnormal behaviors are prevented through the inhibition of ACh level reduction even after the establishment of cocaine dependence. This indicates that a medicinal substance capable of enhancing or disinhibiting ACh actions has therapeutic effects even during the abuse period of cocaine dependence.

### D. Therapeutic Effects of Donepezil on Cocaine Dependence by Enhancement of ACh derived from NAc Cholinergic Cells

The present investigation was conducted to examine whether ACh derived from the NAc cholinergic cells is responsible for cocaine-induced hyperlocomotion. IT-wt and IT-tg mice received intraperitoneal injection of cocaine (10 mg/kg) once a day for 5 days as described in C above. On day 6, the mice were pretreated with saline or donepezil (1 mg/kg) 10 min before intraperitoneal injection of cocaine (10 mg/kg). Their cocaine-induced locomotion was then measured for 10 min (n = 7-11 for each group). Pretreatment with donepezil significantly decreased cocaine-induced abnormal behaviors (locomotor sensitization) in IT-wt group (Fig. 5d, *, P < 0.05). On the contrary, the effects of pretreatment with donepezil were not observed in IT-tg group. These results demonstrate that the action of ChE inhibitors targets ACh derived from the NAc cholinergic cells and the resulting enhancement of ACh in the NAc has the ability to prevent cocaine-induced behavioral abnormality.

### E. Prevention of Relapse of Cocaine-induced Behavioral Abnormality by Donepezil

Drug-induced sensitization caused by continuous daily cocaine administration persists for a long term even after a prolonged period of abstinence from the drug. This is taken seriously as a mechanism of relapse, whereby a drug dependent individual resumes taking a drug after a long-term interruption. The effects of donepezil in blocking relapse of cocaine dependence were examined as follows: Wild-type mice (9-13 weeks) received intraperitoneal injection of cocaine (10 mg/kg) once a day for 6 days and cocaine dependence was established in mice. For 5 days, from day 7 to day 11, mice were withheld from cocaine and were kept in their home cage. On day 12, the mice were pretreated with saline or donepezil (1 mg/kg) 10 min before intraperitoneal injection with cocaine (10 mg/kg). The locomotor activity was then measured for 10 min (n = 6 for each group). In the group pretreated with saline, the abnormal hyperlocomotion was markedly higher on day 12 than on day 1, indicating that the abnormal behavior persisted after a long-term interruption of cocaine administration (Fig. 5e). On the contrary, in the group pretreated with donepezil, no abnormal hyperlocomotion was observed after the cocaine administration on day 12 (Fig. 5e, ***, P < 0.001 when compared with the group pretreated with saline). These results demonstrate that the inhibition of ACh level reduction is effective in prevention of relapse of cocaine-induced abnormal behaviors. These results also demonstrate that a medicinal substance capable of enhancing or disinhibiting the actions of ACh is useful in preventing the relapse during the rehabilitation period of drug dependence.

### EXAMPLE 3 Alleviation of Psychological Alcohol Dependence by Inhibition of ACh Level Reduction

In the Examples above, a medicinal substance capable of inhibiting the reduction of ACh level is effective in the treatment of cocaine or morphine dependence. In the present Example, the investigation was conducted to examine whether a medicinal substance capable of inhibiting the reduction of ACh level is therapeutically effective on alcohol dependence. Wild type mice (9-13 weeks) received daily intraperitoneal injection with 20 %(v/v) ethanol (10 ml/kg) for 3 days as described in EXAMPLE 1A. Mice received saline or donepezil (1 mg/kg) 20 min prior to the administration of ethanol in CPP test (n=7 for each group). As a result, pretreatment with donepezil significantly reduced ethanol-induced CPP (Fig. 7, *, P < 0.05 in comparison with the saline-pretreated group). It has been revealed that the inhibition of ACh level reduction with donepezil can alleviate psychological alcohol dependence. These results indicate that a medicinal substance capable of enhancing or disinhibiting the action of ACh is effective in the treatment of alcohol dependence.

### INDUSTRIAL APPLICABILITY

According to the present invention, an effective means of treating drug dependence is provided for the first time. The pharmaceutical composition of the present invention is useful in the treatment of drug dependence which is accompanied by not only psychological but also physical dependence, and can contribute to solve various social problems related to drug dependence.

## Claims

1. A pharmaceutical composition for use in the treatment of drug dependence which comprises as an active ingredient donepezil or its salt.

2. A pharmaceutical composition for use according to claim 1, wherein said drug dependence is caused by a substance of morphine type, alcohol type or cocaine type.

3. The composition for use according to claim 2, wherein said drug dependence is caused by morphine or cocaine.

4. The composition for use according to claim 2, wherein said drug dependence is caused by alcohol.

5. The composition for use according to any one of claims 2 to 4, wherein the salt of donepezil is donepezil hydrochloride.

6. Use of donepezil or its salt for the preparation of a therapeutic agent for treating drug dependence.

7. Use according to claim 6, wherein said drug dependence is caused by a substance of morphine type, alcohol type or cocaine type.

8. The use according to claim 7, wherein said drug dependence is caused by morphine or cocaine.

9. The use according to claim 7, wherein said drug dependence is caused by alcohol.

10. The use according to any one of claims 7 to 9, wherein the salt of donepezil is donepezil hydrochloride.

11. Donepezil or its salt for use in the treatment of drug dependence.

12. Donepezil or its salt for use according to claim 11, wherein said drug dependence is caused by a substance of morphine type, alcohol type or cocaine type.

13. Donepezil or its salt for use according to claim 12, wherein said drug dependence is caused by morphine or cocaine.

14. Donepezil or its salt for use according to claim 12, wherein said drug dependence is caused by alcohol.

15. Donepezil or its salt for use according to any one of claims 12 to 14, wherein the salt of donepezil is donepezil hydrochloride.

## Patentansprüche

1. Pharmazeutische Zusammensetzung zur Verwendung bei der Behandlung von Drogenabhängigkeit, die als Wirkstoff Donepezil oder ein Salz davon umfasst.

2. Pharmazeutische Zusammensetzung zur Verwendung gemäß Anspruch 1, wobei die Drogenabhängigkeit durch eine Substanz vom Morphintyp, Alkoholtyp oder Kokaintyp verursacht ist.

3. Zusammensetzung zur Verwendung gemäß Anspruch 2, wobei die Drogenabhängigkeit durch Morphin oder Kokain verursacht ist.

4. Zusammensetzung zur Verwendung gemäß Anspruch 2, wobei die Drogenabhängigkeit durch Alkohol verursacht ist.

5. Zusammensetzung zur Verwendung gemäß einem der Ansprüche 2 bis 4, wobei es sich bei dem Salz von Donepezil um Donepezil-Hydrochlorid handelt.

6. Verwendung von Donepezil oder einem Salz davon zur Herstellung eines therapeutischen Mittels zur Behandlung von Drogenabhängigkeit.

7. Verwendung gemäß Anspruch 6, wobei die Drogenabhängigkeit durch eine Substanz vom Morphintyp, Alkoholtyp oder Kokaintyp verursacht ist.

8. Verwendung gemäß Anspruch 7, wobei die Drogenabhängigkeit durch Morphin oder Kokain verursacht ist.

9. Verwendung gemäß Anspruch 7, wobei die Drogenabhängigkeit durch Alkohol verursacht ist.

10. Verwendung gemäß einem der Ansprüche 7 bis 9, wobei es sich bei dem Salz von Donepezil um Donepezil-Hydrochlorid handelt.

11. Donepezil oder Salz davon zur Verwendung bei der Behandlung von Drogenabhängigkeit.

12. Donepezil oder Salz davon zur Verwendung gemäß Anspruch 11, wobei die Drogenabhängigkeit durch eine Substanz vom Morphintyp, Alkoholtyp oder Kokaintyp verursacht ist.

13. Donepezil oder Salz davon zur Verwendung gemäß Anspruch 12, wobei die Drogenabhängigkeit durch Morphin oder Kokain verursacht ist.

14. Donepezil oder Salz davon zur Verwendung gemäß Anspruch 12, wobei die Drogenabhängigkeit durch Alkohol verursacht ist.

15. Donepezil oder Salz davon zur Verwendung gemäß einem der Ansprüche 12 bis 14, wobei es sich bei dem Salz von Donepezil um Donepezil-Hydrochlorid handelt.

## Revendications

1. Composition pharmaceutique destinée à être utilisée dans le traitement de la dépendance aux drogues, comprenant le donépézil ou un sel de celui-ci en tant que principe actif.

2. Composition pharmaceutique destinée à être utilisée selon la revendication 1, dans laquelle ladite dépendance aux drogues est causée par une substance de type morphine, de type alcool ou de type cocaïne.

3. Composition pharmaceutique destinée à être utilisée selon la revendication 2, dans laquelle ladite dépendance aux drogues est causée par la morphine ou la cocaïne.

4. Composition pharmaceutique destinée à être utilisée selon la revendication 2, dans laquelle ladite dépendance aux drogues est causée par l'alcool.

5. Composition pharmaceutique destinée à être utilisée selon l'une quelconque des revendications 2 à 4, dans laquelle le sel du donépézil est l'hydrochlorure de donépézil.

6. Utilisation de donépézil ou d'un sel de celui-ci pour la préparation d'un agent thérapeutique pour le traitement de la dépendance aux drogues.

7. Utilisation selon la revendication 6, dans laquelle ladite dépendance aux drogues est causée par une substance de type morphine, de type alcool ou de type cocaïne.

8. Utilisation selon la revendication 7, dans laquelle ladite dépendance aux drogues est causée par la morphine ou la cocaïne.

9. Utilisation selon la revendication 7, dans laquelle ladite dépendance aux drogues est causée par l'alcool.

10. Utilisation selon l'une quelconque des revendications 7 à 9, dans laquelle le sel du donépézil est l'hydrochlorure de donépézil.

11. Donépézil ou un sel de celui-ci destiné à être utilisée dans le traitement de la dépendance aux drogues.

12. Donépézil ou un sel de celui-ci destiné à être utilisée selon la revendication 11, dans laquelle ladite dépendance aux drogues est causée par une substance de type morphine, de type alcool ou de type cocaïne.

13. Donépézil ou un sel de celui-ci destiné à être utilisée selon la revendication 12, dans laquelle ladite dépendance aux drogues est causée par la morphine ou la cocaïne.

14. Donépézil ou un sel de celui-ci destiné à être utilisée selon la revendication 12, dans laquelle ladite dépendance aux drogues est causée par l'alcool.

15. Donépézil ou un sel de celui-ci destiné à être utilisée selon l'une quelconque des revendications 12 à 14, dans laquelle le sel du donépézil est l'hydrochlorure de donépézil.
